(19) Europäisches Patentamt
European Patent Office
Office européen des brevets

(11) EP 3 162 876 A1

(12) # EUROPEAN PATENT APPLICATION
published in accordance with Art. 153(4) EPC

(43) Date of publication:
**03.05.2017 Bulletin 2017/18**

(21) Application number: **15812507.0**

(22) Date of filing: **24.06.2015**

(51) Int Cl.:
***C10M 133/16*** *(2006.01)* ***C10N 30/00*** *(2006.01)*
***C10N 30/06*** *(2006.01)* ***C10N 40/04*** *(2006.01)*

(86) International application number:
**PCT/JP2015/068172**

(87) International publication number:
**WO 2015/199124 (30.12.2015 Gazette 2015/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **24.06.2014 JP 2014129691**
**24.06.2014 JP 2014129692**
**24.06.2014 JP 2014129696**

(71) Applicant: **Idemitsu Kosan Co., Ltd**
**Tokyo 100-8321 (JP)**

(72) Inventors:
- **KOSHIMA, Hiroaki**
**Sodegaura-shi**
**Chiba 299-0293 (JP)**
- **DESHIMARU. Junichi**
**Ichihara-shi**
**Chiba 299-0193 (JP)**
- **IWAI, Toshiaki**
**Ichihara-shi**
**Chiba 299-0193 (JP)**

(74) Representative: **Hoffmann Eitle**
**Patent- und Rechtsanwälte PartmbB**
**Arabellastraße 30**
**81925 München (DE)**

(54) **LUBRICATION OIL ADDITIVE AND METHOD FOR MANUFACTURING SAME, AND LUBRICATION OIL COMPOSITION USING SAME**

(57) Through a lubricating oil additive including an amide compound represented by the following general formula (1) and a lubricating oil composition using the same, a lubricating oil additive and a lubricating oil composition exhibiting not only a high intermetal friction coefficient but also excellent intermetal friction-coefficient/slipping-velocity characteristics are provided.

$$R^a\text{-CO-(NH-L}^1)_n\text{-NR}^bR^c \quad (1)$$

In the formula, $R^a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group.



Printed by Jouve, 75001 PARIS (FR)

## Description

TECHNICAL FIELD

The present invention relates to a lubricating oil additive and a method for producing the same, and a lubricating oil composition using the same.

BACKGROUND ART

In order to address the great issue of improving fuel efficiency in view of concern on global environment, in automobiles, a mounting rate of a continuously variable transmission (CVT) with high efficiency is increasing.

Examples of the CVT include a metal-made push-belt type and a chain type. Since the power is transmitted via a friction between a pulley and a belt or between a pulley and a chain, a large force is applied in order to restrain a slippage therebetween. A CVT fluid is used for lubrication between the belt and the pulley or between the chain and the pulley. Since reduction in a force for forcing one of these members to the other results in improvement of fuel efficiency, a high intermetal friction coefficient between the members is required for the CVT fluid.

SUMMARY OF INVENTION

TECHNICAL PROBLEM

As the CVT fluid, those capable of increasing the intermetal friction coefficient between the members are required. However, following an increase of the intermetal friction coefficient, a vibration or noise between the pulley and the belt or between the pulley and the chain becomes often problematic. From the viewpoint of inhibiting this problem, the CTV fluid is also demanded to exhibit not only a high intermetal friction coefficient between the pulley and the belt or between the pulley and the chain but also excellent intermetal friction-coefficient/slipping-velocity characteristics.

In view of the aforementioned circumstances, the present invention has been made, and an object thereof is to provide a lubricating oil additive and a lubricating oil composition exhibiting not only a high intermetal friction coefficient but also excellent intermetal friction-coefficient/slipping-velocity characteristics.

SOLUTION TO PROBLEM

The present inventors made extensive and intensive investigations. As a result, it has been found that the aforementioned problem can be solved by an additive containing a specified amide compound.

Specifically, the present invention provides the following.

1. A lubricating oil additive including an amide compound represented by the following general formula (1):

$$R^a\text{-CO-(NH-}L^1)_n\text{-NR}^bR^c \qquad (1)$$

wherein $R^a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group.

2. A lubricating oil composition including the lubricating oil additive as set forth in the above item 1.

3. A method for producing the lubricating oil additive as set forth in the above item 1, including a step of allowing a fatty acid represented by the following general formula (5) and a polyalkylenepolyamine represented by the following general formula (6) to react with each other, the polyalkylenepolyamine being used in an amount of 1 mol or more based on 1 mol of the fatty acid:

$$R^a\text{-COOH} \qquad (5)$$

$$H\text{-(NH-}L^1)_n\text{-NR}^bR^c \qquad (6)$$

wherein $R^a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group.

ADVANTAGEOUS EFFECTS OF INVENTION

In accordance with the present invention, it is possible to provide a lubricating oil additive and a lubricating oil composition exhibiting not only a high intermetal friction coefficient but also excellent intermetal friction-coefficient/slipping-velocity characteristics.

DESCRIPTION OF EMBODIMENTS

The present invention is hereunder described in detail with reference to embodiments.

<Lubricating Oil Additive>

The lubricating oil additive of the present invention includes an amide compound represented by the following general formula (1).

$$R^a\text{-CO-(NH-}L^1)_n\text{-NR}^bR^c \qquad (1)$$

In the aforementioned formula, $R^a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group. Here, what $-NR^bR^c$ is a primary amino group refers to the matter that both $R^b$ and $R^c$ are a hydrogen atom, and what $-NR^bR^c$ is a piperazyl group refers to the matter that $R^b$ and $R^c$ are bonded to each other together with a N atom to form a piperazyl group.

Suitable embodiments of the lubricating oil additive including the amide compound represented by the general formula (1) are the following three (first to third lubricating oil additives of the present invention), and the lubricating oil additive of the present invention is described in detail with reference to these embodiments.

(First Lubricating Oil Additive of the Present Invention)

The first lubricating oil additive according to the present invention (hereinafter sometimes referred to "first lubricating oil additive") contains an amide compound represented by the following general formula (2).

$$R^1\text{-CO-(NH-}L^1)_n\text{-NH}_2 \qquad (2)$$

In the aforementioned formula, $R^1$ is a straight-chain alkyl group having 9 to 21 carbon atoms or a straight-chain alkenyl group having 9 to 21 carbon atoms $L^1$ is an alkylene group having 1 to 6 carbon atoms; and n is an integer of 2 to 4.

In order to exhibit effectively the effects in the present invention, the carbon number in $R^1$ is preferably 9 to 17. In addition, the carbon number in $L^1$ is preferably 1 to 4, and more preferably 1 to 3. Furthermore, n is preferably 2 to 3.

In the general formula (2), examples of the straight-chain alkyl group represented by $R^1$ include an n-nonyl group, an n-decyl group, an n-undecyl group, an n-dodecyl group, an n-tridecyl group, an n-tetradecyl group, an n-pentadecyl group, an hexadecyl group, an n-heptadecyl group, an n-octadecyl group, an n-nonadecyl group, an n-eicosyl group, an n-heneicosyl group, and the like. Examples of the straight-chain alkenyl group represented by $R^1$ include various nonenyl groups, decenyl groups, undecenyl groups, dodecenyl groups, tridecenyl groups, tetradecenyl groups, pentadecenyl groups, hexadecenyl groups, heptadecenyl groups, octadecenyl groups, nonadecenyl groups, eicosenyl groups, and heneicosenyl groups, and the like.

In the general formula (2), examples of the alkylene group represented by $L^1$ include a methylene group, an ethylene group, a trimethylene group, a tetramethylene group, a pentamethylene group, a hexamethylene group, and the like. From the viewpoint that the hydrocarbon chain length between nitrogen and nitrogen in the compound is short, so that the intermetal friction coefficient can be made low, the alkylene group represented by $L^1$ is preferably an ethylene group or a trimethylene group.

In the general formula (2), n is an integer of 2 to 4, and from the viewpoint of obtaining excellent intermetal friction-coefficient/slipping-velocity characteristics, n is preferably 2 or 3, and most preferably 2.

From the viewpoint of allowing the first lubricating oil additive to exhibit the aforementioned effects through addition of a small amount thereof to the lubricating oil, the content of the amide compound represented by the general formula (2) is preferably 50% by mass or more, more preferably 70% by mass or more, sill more preferably 80% by mass or more, and especially preferably 90% by mass or more.

The first lubricating oil additive is used preferably for a driving system oil, and more preferably for a chain type

CVT or belt type CVT.

(Second Lubricating Oil Additive of the Present Invention)

The second lubricating oil additive according to the present invention (hereinafter sometimes referred to "second lubricating oil additive") contains an amide compound represented by the following general formula (3).

$$R^2\text{-CO-(NH-}L^1)_n\text{-}NH_2 \qquad (3)$$

In the aforementioned formula, $R^2$ is a branched-chain alkyl group having 9 to 21 carbon atoms or a branched-chain alkenyl group having 9 to 21 carbon atoms; $L^1$ is an alkylene group having 1 to 6 carbon atoms; and n is an integer of 2 to 4, provided that the number of branched chains which each of the alkyl group and the alkenyl group has is 3 or less, and that the branched chain is a methyl group.

In order to exhibit effectively the effects in the present invention, the carbon number in $R^2$ is preferably 9 to 17. In addition, the carbon number in $L^1$ is preferably 1 to 4, and more preferably 1 to 3. Furthermore, n is preferably 2 to 3.

In the general formula (3), examples of the branched-chain alkyl group represented by $R^2$ include those selected from a branched nonyl group, a branched decyl group, a branched undecyl group, a branched dodecyl group, a branched tridecyl group, a branched tetradecyl group, a branched pentadecyl group, a branched hexadecyl group, a branched heptadecyl group, a branched octadecyl group, a branched nonadecyl group, a branched eicosyl group, and a branched heneicosyl group, in which the number of branched chains is 3 or less, and the branched chain is a methyl group, and specifically, a methyl-n-hexadecyl group and the like are exemplified. Examples of the branched alkenyl group represented by $R^2$ include those selected from a branched nonenyl group, a branched decenyl group, a branched undecenyl group, a branched dodecenyl group, a branched tridecenyl group, a branched tetradecenyl group, a branched pentadecenyl group, a branched hexadecenyl group, a branched heptadecenyl group, a branched octadecenyl group, a branched nonadecenyl group, a branched eicosenyl group, a branched heneicosenyl group, and the like, in which the number of branched chains is 3 or less, and the branched chain is a methyl group.

In the general formula (3), examples of the alkylene group represented by $L^1$ include a methylene group, an ethylene group, a trimethylene group, a tetramethylene group, a pentamethylene group, and a hexamethylene group. From the viewpoint that the hydrocarbon chain length between nitrogen and nitrogen in the compound is short, so that the intermetal friction coefficient can be made low, the alkylene group represented by $L^1$ is preferably an ethylene group or a trimethylene group.

In the general formula (3), n is an integer of 2 to 4, and from the viewpoint of obtaining excellent intermetal friction-coefficient/slipping-velocity characteristics, n is preferably 2 or 3, and most preferably 2.

From the viewpoint of allowing the second lubricating oil additive to exhibit the aforementioned effects through addition of a small amount thereof to the lubricating oil, the content of the amide compound represented by the general formula (3) is preferably 50% by mass or more, more preferably 70% by mass or more, sill more preferably 80% by mass or more, and especially preferably 90% by mass or more.

The second lubricating oil additive is used preferably for a driving system oil, and more preferably for a chain type CVT or belt type CVT.

(Third Lubricating Oil Additive of the Present Invention)

The third lubricating oil additive according to the present invention (hereinafter sometimes referred to "third lubricating oil additive") contains an amide compound represented by the following general formula (4).

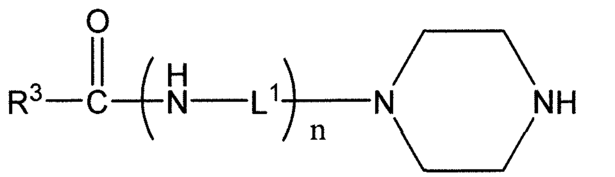

(4)

In the aforementioned formula, $R^3$ is a straight-chain alkyl group having 8 to 22 carbon atoms, a branched-chain alkyl group having 8 to 22 carbon atoms, a straight-chain alkenyl group having 8 to 22 carbon atoms, or a branched-chain alkenyl group having 8 to 22 carbon atoms; $L^1$ is an alkylene group having 1 to 6 carbon atoms; and n is an integer of 1 to 4, provided that the number of branched chains which each of the branched-chain alkyl group and the branched-chain alkenyl group has is 3 or less, and that the branched chain is a methyl group.

In order to exhibit effectively the effects in the present invention, the carbon number in $R^3$ is preferably 9 to 21,

and more preferably 9 to 17. In addition, the carbon number in $L^1$ is preferably 1 to 4, and more preferably 1 to 3. Furthermore, n is preferably 1 to 3, more preferably 1 to 2.

In the general formula (4), examples of the straight-chain alkyl group represented by $R^3$ include an n-nonyl group, an n-decyl group, an n-undecyl group, an n-dodecyl group, an n-tridecyl group, an n-tetradecyl group, an n-pentadecyl group, an hexadecyl group, an n-heptadecyl group, an n-octadecyl group, an n-nonadecyl group, an n-eicosyl group, an n-heneicosyl group, and the like. Examples of the straight-chain alkenyl group represented by $R^3$ include various nonenyl groups, decenyl groups, undecenyl groups, dodecenyl groups, tridecenyl groups, tetradecenyl groups, pentadecenyl groups, hexadecenyl groups, heptadecenyl groups, octadecenyl groups, nonadecenyl groups, eicosenyl groups, and heneicosenyl groups, and the like.

Examples of the branched-chain alkyl group represented by $R^3$ include those selected from a branched nonyl group, a branched decyl group, a branched undecyl group, a branched dodecyl group, a branched tridecyl group, a branched tetradecyl group, a branched pentadecyl group, a branched hexadecyl group, a branched heptadecyl group, a branched octadecyl group, a branched nonadecyl group, a branched eicosyl group, and a branched heneicosyl group, in which the number of branched chains is 3 or less, and the branched chain is a methyl group, and specifically, a methyl-n-hexadecyl group and the like are exemplified. Examples of the branched alkenyl group represented by $R^3$ include those selected from a branched nonenyl group, a branched decenyl group, a branched undecenyl group, a branched dodecenyl group, a branched tridecenyl group, a branched tetradecenyl group, a branched pentadecenyl group, a branched hexadecenyl group, a branched heptadecenyl group, a branched octadecenyl group, a branched nonadecenyl group, a branched eicosenyl group, a branched heneicosenyl group, and the like, in which the number of branched chains is 3 or less, and the branched chain is a methyl group.

In the general formula (4), examples of the alkylene group represented by $L^1$ include a methylene group, an ethylene group, a trimethylene group, a tetramethylene group, a pentamethylene group, and a hexamethylene group. From the viewpoint that the hydrocarbon chain length between nitrogen and nitrogen in the compound is short, so that the intermetal friction coefficient can be made low, the alkylene group represented by $L^1$ is preferably an ethylene group or a trimethylene group.

In the general formula (4), n is an integer of 1 to 4, and from the viewpoint of obtaining excellent intermetal friction-coefficient/slipping-velocity characteristics, n is preferably 1 or 2, and most preferably 1.

From the viewpoint of allowing the third lubricating oil additive to exhibit the aforementioned effects through addition of a small amount thereof to the lubricating oil, the content of the amide compound represented by the general formula (4) is preferably 50% by mass or more, more preferably 70% by mass or more, sill more preferably 80% by mass or more, and especially preferably 90% by mass or more.

The third lubricating oil additive is used preferably for a driving system oil, and more preferably for a chain type CVT or belt type CVT.

<Production Method of Lubricating Oil Additive>

The production method of a lubricating oil additive of the present invention includes a step of allowing a fatty acid represented by the following general formula (5) and a polyalkylenepolyamine represented by the following general formula (6) to react with each other, the polyalkylenepolyamine being used in an amount of 1 mol or more based on 1 mol of the fatty acid.

$$R^a\text{-COOH} \qquad (5)$$

$$H\text{-}(NH\text{-}L^1)_n\text{-}NR^bR^c \qquad (6)$$

In the aforementioned formulae, $R^a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group.

Suitable embodiments of the production method of a lubricating oil additive of the present invention are the following three (first to third production methods of a lubricating oil additive of the present invention), and the production method of a lubricating oil additive of the present invention is described in detail with reference to these embodiments.

(First Production Method of Lubricating Oil Additive)

The first lubricating oil additive can be produced by allowing a fatty acid represented by the following general formula (7) and a polyalkylenepolyamine represented by the following general formula (8) to react with each other, the polyalkylenepolyamine being used in an amount of 1 mol or more based on 1 mol of the fatty acid, and the ratio of the

polyalkylenepolyamine based on 1 mol of the fatty acid is preferably 2 moles or more, and especially preferably 3 moles or more. An upper limit of the polyalkylenepolyamine is about 10 moles.

$R^1$-COOH (7)

H-(NH-$L^1$)$_n$-$NH_2$ (8)

In the aforementioned formulae, $R^1$ is a straight-chain alkyl group having 9 to 21 carbon atoms or a straight-chain alkenyl group having 9 to 21 carbon atom; $L^1$ is an alkylene group having 1 to 6 carbon atoms; and n is an integer of 2 to 4.
Details of $R^1$ in the general formula (7) are the same as those in $R^1$ in the general formula (2).
Details of $L^1$ and n in the general formula (8) are the same as those in $L^1$ and n in the general formula (2), respectively.
Though the method of allowing the polyalkylenepolyamine and the fatty acid to react with each other is not particularly limited, for example, a method in which the polyalkylenepolyamine is charged into a reactor, to which is then added dropwise the fatty acid, is preferably adopted.
A reaction temperature on the occasion of allowing the polyalkylenepolyamine and the fatty acid to react with each other is preferably 100 to 250°C, more preferably 150 to 220°C, and still more preferably 170 to 200°C.
Though a reaction pressure on the occasion of allowing the polyalkylenepolyamine and the fatty acid to react with each other may be atmospheric pressure, reduced pressure, or elevated pressure, a pressure range is preferably 0.1026 to 1.1143 MPa, more preferably 0.1146 to 0.6078 MPa, and still more preferably 0.1925 to 0.3039 MPa in terms of an absolute pressure.

(Second Production Method of Lubricating Oil Additive)

The second lubricating oil additive can be produced by allowing a fatty acid represented by the following general formula (9) and a polyalkylenepolyamine represented by the following general formula (10) to react with each other, the polyalkylenepolyamine being used in an amount of 1 mol or more based on 1 mol of the fatty acid, and the ratio of the polyalkylenepolyamine based on 1 mol of the fatty acid is preferably 2 moles or more, and especially preferably 3 moles or more. An upper limit of the polyalkylenepolyamine is about 10 moles.

$R^2$-COOH (9)

H-(NH-$L^1$)$_n$-$NH_2$ (10)

In the aforementioned formulae, $R^2$ is a branched-chain alkyl group having 9 to 21 carbon atoms or a branched-chain alkenyl group having 9 to 21 carbon atoms; $L^1$ is an alkylene group having 1 to 6 carbon atoms; and n is an integer of 2 to 4, provided that the number of branched chains which each of the alkyl group and the alkenyl group has is 3 or less, and that the branched chain is a methyl group.
Details of $R^2$ in the general formula (9) are the same as those in $R^2$ in the general formula (3).
Details of $L^1$ and n in the general formula (10) are the same as those in $L^1$ and n in the general formula (3), respectively.
Though the method of allowing the polyalkylenepolyamine and the fatty acid to react with each other is not particularly limited, for example, a method in which the polyalkylenepolyamine is charged into a reactor, to which is then added dropwise the fatty acid, is preferably adopted.
A reaction temperature on the occasion of allowing the polyalkylenepolyamine and the fatty acid to react with each other is preferably 100 to 250°C, more preferably 150 to 220°C, and still more preferably 170 to 200°C.
Though a reaction pressure on the occasion of allowing the polyalkylenepolyamine and the fatty acid to react with each other may be atmospheric pressure, reduced pressure, or elevated pressure, a pressure range is preferably 0.1026 to 1.1143 MPa, more preferably 0.1146 to 0.6078 MPa, and still more preferably 0.1925 to 0.3039 MPa in terms of an absolute pressure.

(Third Production Method of Lubricating Oil Additive)

The third lubricating oil additive can be produced by allowing a fatty acid represented by the following general formula (11) and an amine compound represented by the following general formula (12) to react with each other, the amine compound being used in an amount of 1 mol or more based on 1 mol of the fatty acid, and the ratio of the amine compound based on 1 mol of the fatty acid is preferably 2 moles or more, and especially preferably 3 moles or more.

An upper limit of the polyalkylenepolyamine is about 10 moles.

$R^3$-COOH (11)

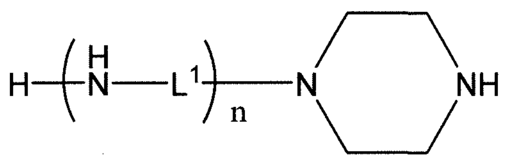

(12)

In the aforementioned formulae, $R^3$ is a straight-chain alkyl group having 8 to 22 carbon atoms, a branched-chain alkyl group having 8 to 22 carbon atoms, a straight-chain alkenyl group having 8 to 22 carbon atoms, or a branched-chain alkenyl group having 8 to 22 carbon atoms; $L^1$ is an alkylene group having 1 to 6 carbon atoms; and n is an integer of 1 to 4, provided that the number of branched chains which each of the branched-chain alkyl group and the branched-chain alkenyl group has is 3 or less, and that the branched chain is a methyl group.

Details of $R^3$ in the general formula (11) are the same as those in $R^3$ in the general formula (4).

Details of $L^1$ and n in the general formula (12) are the same as those in $L^1$ and n in the general formula (4), respectively.

Though the method of allowing the amine compound and the fatty acid to react with each other is not particularly limited, for example, a method in which the amine compound is charged into a reactor, to which is then added dropwise the fatty acid, is preferably adopted.

A reaction temperature on the occasion of allowing the amine compound and the fatty acid to react with each other is preferably 100 to 250°C, more preferably 150 to 220°C, and still more preferably 170 to 200°C.

Though a reaction pressure on the occasion of allowing the amine compound and the fatty acid to react with each other may be atmospheric pressure, reduced pressure, or elevated pressure, a pressure range is preferably 0.1026 to 1.1143 MPa, more preferably 0.1146 to 0.6078 MPa, and still more preferably 0.1925 to 0.3039 MPa in terms of an absolute pressure.

<Lubricating Oil Composition>

The present invention also provides a lubricating oil composition containing a lubricant base oil and the aforementioned lubricating oil additive of the present invention.

The content of the lubricating oil additive in the lubricating oil composition of the present invention is preferably 0.05 mass% or more, more preferably 0.1 mass% by more, and still more preferably 0.2 mass% or more. In addition, the content of the lubricating oil additive is preferably 5 mass% or less, more preferably 2 mass% or less, and still more preferably 1 mass% or more. When the content is too small as compared with the aforementioned suitable range, there might be a case where sufficient intermetal friction-coefficient/slipping-velocity characteristics are not obtained; whereas when the content is too large as compared with the aforementioned suitable range, there might be a case where the intermetal friction-coefficient/slipping-velocity characteristics peak, so that not only no further effect is obtained, but also the intermetal friction coefficient is lowered.

As the lubricant base oil that is used in the present invention, one or more selected from a mineral oil and a synthetic oil can be used.

Though such mineral oil and synthetic oil are not particularly limited, those having a kinematic viscosity at 100°C of 0.5 $mm^2/s$ or more and 50 $mm^2/s$ or less are preferred, and those having a kinematic viscosity at 100°C of 1 $mm^2/s$ or more and 15 $mm^2/s$ or less are more preferred. When the kinematic viscosity is too low as compared with the aforementioned suitable range, there might be a case where an oil film is liable to be hardly formed, so that the base oil is inferior in abrasion resistance; whereas when the kinematic viscosity is too high as compared with the aforementioned suitable range, there might be a case where the viscous resistance increases, so that the base oil is inferior in improvement of fuel efficiency.

Though a pour point that is an indicator of low-temperature fluidity of the lubricant base oil is not particularly limited, it is preferably -10°C or lower, more preferably -15°C or lower, and still more preferably -40°C or lower. When the pour point falls within the aforementioned range, the base oil is excellent in startability at low temperatures and thoroughly adaptable for use in a cold district and the like.

Furthermore, a viscosity index of the lubricant base oil is preferably 100 or more, more preferably 110 or more, and still more preferably 120 or more. When the viscosity index falls within the aforementioned range, the uniform viscosity can be maintained over a wide temperature region, and deterioration of startability to be caused due to a lowering of fluidity at low temperatures, or the like can be prevented from occurring.

Examples of the mineral oil include a naphthenic mineral oil, a paraffinic mineral oil, a GTL oil, and the like. Specifically, there can be exemplified a light neutral oil, a medium neutral oil, a heavy neutral oil, and bright stock, each of which is obtained through solvent refining or hydrogenation refining, and the like.

Meanwhile, examples of the synthetic oil include polybutene or hydrides thereof, poly-$\alpha$-olefins (e.g., a 1-octene oligomer, a 1-decene oligomer, etc.), $\alpha$-olefin copolymers, alkylbenzenes, polyol esters, dibasic acid esters, polyoxyalkylene glycols, polyoxyalkylene glycol esters, polyoxyalkylene glycol ethers, hindered esters, silicone oils, and the like.

The content of the lubricant base oil in the whole amount of the lubricating oil composition of the present invention is preferably 50 mass% or more, more preferably 60 mass% or more, and still more preferably 70 mass% or more. When the content of the lubricant base oil is below the aforementioned range, there might be a case where the oil film holding is hardly achieved, so that the lubricity or abrasion resistance is lowered.

The lubricating oil composition according to the present invention may contain other additives. Examples thereof include a detergent dispersant, an antioxidant, a rust inhibitor, an anti-wear agent, a friction modifier, a friction reliever, a pour-point depressant, a defoaming agent, an extreme pressure agent, a viscosity index improver, a thickener, and the like.

The lubricating oil composition of the present invention is applicable to, for example, an automatic transmission, a continuously variable transmission, a manual transmission, a gear wheel, and the like, and in particular, it is suitable for a continuously variable transmission, and especially suitable for a chain type CVT or belt type CVT.

EXAMPLES

Next, the present invention is described in more detail with reference to Examples, but it should be construed that the present invention is by no means limited by these Examples.

<Examples Regarding First Lubricating Oil Additive>

Production Example A1 [Synthesis of N-[2-[(2-Aminoethyl)amino]ethyl]decylamide]

In a 200-mL four-neck round bottom flask, 103.2 g (1.0 mol) of diethylenetriamine was charged and subjected to temperature rise to about 90°C in a nitrogen gas stream. Subsequently, 34.5 g (0.2 moles) of decanoic acid was added dropwise in the flask. After stirring for 30 minutes such that the system became uniform, the resultant was subjected to temperature rise to 200°C and then subjected to reaction at atmospheric pressure (the same as in the following Production Examples) for 2 hours. Furthermore, the contents of the flask were subjected to temperature drop to 150°C, and the excessive diethylenetriamine and residual water were distilled off while gradually evacuating the inside of the flask, thereby obtaining Reaction Product A1 containing N-[2-[(2-aminoethyl)amino]ethyl]decylamide ($C_9H_{19}CO-NH-C_2H_4-NH-C_2H_4-NH_2$) as a main ingredient (90 mass%, hereinafter the same). The presence of the amide compound was confirmed by subjecting the Reaction Product A1 to $^1H$-NMR. $^1H$-NMR spectra as measured with respect to the Reaction Product A1 are shown below.

$^1H$-NMR Spectra ($CDCl_3$, 0.03% TMS)

| $\delta$ Value (ppm) | Splitting | Assignment | Integration ratio (theoretical ratio) |
|---|---|---|---|
| 0.8 to 0.9 | t | H of methyl of decyl group | 3.2 (3) |
| 1.2 to 1.4 | s | H of methylene of decyl group | 14.1 (14) |
| | | H of terminal amino group of diethylenetriamine | |
| 1.5 to 1.7 | bs | H of $\beta$-position methylene of decyl group | 3.2 (3) |
| | | H of intermediate amino group of diethylenetriamine | |
| 2.1 to 2.3 | m | H of $\alpha$-position methylene of decyl group | 2.0 (2) |
| 2.8 to 3.4 | m | H of methylene of diethylenetriamine | 5.7 (6) |
| 3.7 to 3.8 | m | H of amide-side methylene of diethylenetriamine | 1.8 (2) |
| 5.6 to 5.7 | s | H of amide group | 0.7 (1) |

Production Example A2 [Synthesis of N-[2-[(2-Aminoethyl)amino]ethyl]dodecylamide]

The reaction was performed in the same manner as in Production Example A1, except for using 40.0 g (0.2 moles) of dodecanoic acid in place of 34.5 g (0.2 moles) of the decanoic acid, thereby obtaining Reaction Product A2 containing N-[2-[(2-aminoethyl)amino]ethyl]dodecylamide ($C_{11}H_{23}CO-NH-C_2H_4-NH-C_2H_4-NH_2$) as a main ingredient.

The presence of the amide compound was confirmed by means of $^1$H-NMR.

Production Example A3 [Synthesis of N-[2-[(2-Aminoethyl)amino]ethyl]tetradecylamide]

The reaction was performed in the same manner as in Production Example A1, except for using 45.7 g (0.2 moles) of tetradecanoic acid in place of 34.5 g (0.2 moles) of the decanoic acid, thereby obtaining Reaction Product A3 containing N-[2-[(2-aminoethyl)amino]ethyl]tetradecylamide ($C_{13}H_{27}CO$-NH-$C_2H_4$-NH-$C_2H_4$-$NH_2$) as a main ingredient. The presence of the amide compound was confirmed by means of $^1$H-NMR.

Production Example A4 [Synthesis of N-[2-[(2-Aminoethyl)amino]ethyl]hexadecylamide]

The reaction was performed in the same manner as in Production Example A1, except for using 82.6 g (0.8 moles) of diethylenetriamine in place of 103.2 g (1.0 mol) of the diethylenetriamine and 51.3 g (0.2 moles) of hexadecanoic acid in place of 34.5 g (0.2 moles) of the decanoic acid, respectively, thereby obtaining Reaction Product A4 containing N-[2-[(2-aminoethyl)amino]ethyl]hexadecylamide ($C_{15}H_{31}CO$-NH-$C_2H_4$-NH-$C_2H_4$-$NH_2$) as a main ingredient. The presence of the amide compound was confirmed by means of $^1$H-NMR.

Production Example A5 [Synthesis of N-[2-[(2-Aminoethyl)amino]ethyl]stearylamide]

The reaction was performed in the same manner as in Production Example A4, except for using 56.9 g (0.2 moles) of stearic acid in place of 51.3 g (0.2 moles) of the hexadecanoic acid, thereby obtaining Reaction Product A5 containing N-[2-[(2-aminoethyl)amino]ethyl]stearylamide ($C_{17}H_{35}CO$-NH-$C_2H_4$-NH-$C_2H_4$-$NH_2$) as a main ingredient. The presence of the amide compound was confirmed by means of $^1$H-NMR.

Production Example A6 [Synthesis of N-[2-[(2-Aminoethyl)amino]ethyl]oleylamide]

The reaction was performed in the same manner as in Production Example A4, except for using 56.5 g (0.2 moles) of oleic acid in place of 51.3 g (0.2 moles) of the hexadecanoic acid, thereby obtaining Reaction Product A6 containing N-[2-[(2-aminoethyl)amino]ethyl]oleylamide ($C_{17}H_{33}CO$-NH-$C_2H_4$-NH-$C_2H_4$-$NH_2$) as a main ingredient. The presence of the amide compound was confirmed by subjecting the Reaction Product A6 to $^1$H-NMR. $^1$H-NMR spectra as measured with respect to the Reaction Product A6 are shown below.

$^1$H-NMR Spectra ($CDCl_3$, 0.03% TMS)

| δ Value (ppm) | Splitting | Assignment | Integration ratio (theoretical ratio) |
|---|---|---|---|
| 0.8 to 0.9 | t | H of methyl of oleyl group | 3.2 (3) |
| 1.2 to 1.4 | m | H of methylene of oleyl group | 21.2 (22) |
| | | H of terminal amino group of diethylenetriamine | |
| 1.5 to 1.7 | bs | H of β-position methylene of oleyl group | 2.9 (3) |
| | | H of intermediate amino group of diethylenetriamine | |
| 2.0 | bs | H of methylene adjacent to olefin of oleyl group | 4.0 (4) |
| 2.1 to 2.3 | m | H of α-position methylene of oleyl group | 2.0 (2) |
| 2.8 to 3.4 | m | H of methylene of diethylenetriamine | 5.8 (6) |
| 3.7 | m | H of amide-side methylene of diethylenetriamine | 1.8 (2) |
| 5.3 to 5.4 | s | H of olefin of oleyl group | 2.1 (2) |
| 5.7 to 5.8 | s | H of amide group | 0.6 (1) |

Production Example A7 [Synthesis of N-[2-[(2-Aminoethyl)amino]ethyl]erucylamide]

The reaction was performed in the same manner as in Production Example A4, except for using 67.7 g (0.2 moles) of erucic acid in place of 51.3 g (0.2 moles) of the hexadecanoic acid, thereby obtaining Reaction Product A7 containing N-[2-[(2-aminoethyl)amino]ethyl]erucylamide ($C_{21}H_{41}CO$-NH-$C_2H_4$-NH-$C_2H_4$-$NH_2$) as a main ingredient. The presence of the amide compound was confirmed by means of $^1$H-NMR.

Production Example A8 [Synthesis of N-[2-[[2-[(2-Aminoethyl)amino]ethyl]amino]ethyl]decylamide]

The reaction was performed in the same manner as in Production Example A1, except for using 146.2 g (1.0 mol) of triethylenetetramine in place of 103.2 g (1.0 mol) of the diethylenetriamine, thereby obtaining Reaction Product A8 containing N- [2-[[2-[(2-aminoethyl)amino]ethyl]amino]ethyl]decylamide ($C_9H_{19}CO-NH-C_2H_4-NH-C_2H_4-NH-C_2H_4-NH_2$) as a main ingredient. The presence of the amide compound was confirmed by means of $^1H$-NMR.

Production Example A9 [Synthesis of N- [2- [[2- [(2-Aminoethyl)amino]ethyl]amino]ethyl]oleylamide]

The reaction was performed in the same manner as in Production Example A4, except for using 117.0 g (0.8 moles) of triethylenetetramine in place of 82.6 g (0.8 moles) of the diethylenetriamine and 56.5 g (0.2 moles) of oleic acid in place of 51.3 g (0.2 moles) of the hexadecanoic acid, respectively, thereby obtaining Reaction Product A9 containing N-[2-[[2-[(2-aminoethyl)amino]ethyl]amino]ethyl]oleylamide ($C_{17}H_{33}CO-NH-C_2H_4-NH-C_2H_4-NH-C_2H_4-NH_2$) as a main ingredient. The synthesis of the compound was confirmed by means of $^1H$-NMR.

Production Example A10 [Synthesis of N-[2-[[2-[[2-[(2-Aminoethyl)amino]ethyl]amino)ethyl]amino]ethyl]oleylamide]

The reaction was performed in the same manner as in Production Example A4, except for using 151.4 g (0.8 moles) of tetraethylenepentamine in place of 82.6 g (0.8 moles) of the diethylenetriamine and 56.5 g (0.2 moles) of oleic acid in place of 51.3 g (0.2 moles) of the hexadecanoic acid, respectively, thereby obtaining Reaction Product A10 containing N-[2-[[2-[[2-[(2-Aminoethyl)amino]ethyl]amino)ethyl]amino]ethyl]oleylamide ($C_{17}H_{33}CO-NH-C_2H_4-NH-C_2H_4-NH-C_2H_4-NH-C_2H_4-NH_2$) as a main ingredient. The presence of the amide compound was confirmed by means of $^1H$-NMR.

Production Example A11 [Synthesis of N-[2-[(2-Aminoethyl)amino]ethyl]oleylamide]

The reaction was performed in the same manner as in Production Example A6, except for using 61.9 g (0.6 moles) of diethylenetriamine in place of 82.6 g (0.8 moles) of the diethylenetriamine, thereby obtaining Reaction Product A11 containing N-[2-[(2-aminoethyl)amino]ethyl]oleylamide ($C_{17}H_{33}CO-NH-C_2H_4-NH-C_2H_4-NH_2$) as a main ingredient. The composition of the Reaction Product A11 was analyzed by means of the following liquid chromatography (LC) and mass analysis (MS). As a result, a proportion of N-[2-[(2-aminoethyl)amino]ethyl]oleylamide to bis(2-(oleylamido)ethyl)amine was found to be 91/9 (mass ratio).

[LC Measurement Conditions]

- Sample concentration: 1,000 ppm/THF
- Apparatus: Agilent 1200 Series HPLC
- Column^ L Column-2 (2.1 × 150 mm, 3 um)
- Mobile phase: A) 0.1% HCOOH + 0.1% $HCOONH_4/CH_3CN$ = 20/80, B) THF
- Gradient program: 0 min. B) 20% → 10 min. B) 100% (post time: 7 min.)
- Flow rate: 0.3 mL/min.
- Injection volume: 1.5 μL
- Detector: Evaporative light scattering detector (ELSD)

[MS Measurement Conditions]

- Apparatus: Linear ion trap mass spectrometer LXQ, manufactured by Thermo Fisher Scientific Inc.
- Column: L Column-2 (2.1 × 150 mm, 3 μm)
- Mobile phase: A) 0.1% HCOOH + 0.1% $HCOONH_4/CH_3CN$ = 20/80, B) THF
- Gradient program: 0 min. B) 20% → 10 min. B) 100% (post time: 7 min.)
- Flow rate: 0.3 mL/min.
- Injection volume: 1.5 μL
- Ionization: HESI

Production Example A12 [Synthesis of N-[2-[(2-Aminoethyl)amino]ethyl]oleylamide]

The reaction was performed in the same manner as in Production Example A6, except for using 41.3 g (0.4 moles) of diethylenetriamine in place of 82.6 g (0.8 moles) of the diethylenetriamine, thereby obtaining Reaction Product A12 containing N-[2-[(2-aminoethyl)amino]ethyl]oleylamide ($C_{17}H_{33}CO$-NH-$C_2H_4$-NH-$C_2H_4$-$NH_2$) as a main ingredient. The composition of the Reaction Product A12 was analyzed by means of liquid chromatography (LC). As a result, a proportion of N-[2-[(2-aminoethyl)amino]ethyl]oleylamide to bis(2-(oleylamido)ethyl)amine was found to be 83/17 (mass ratio).

The liquid chromatography was carried out under the same measurement conditions as in Production Example A11.

Production Example A13 [Synthesis of N-[2-[(2-Aminoethyl)amino]ethyl]oleylamide]

The reaction was performed in the same manner as in Production Example A6, except for using 20.6 g (0.2 moles) of diethylenetriamine in place of 82.6 g (0.8 moles) of the diethylenetriamine, thereby obtaining Reaction Product A13 containing N-[2-[(2-aminoethyl)amino]ethyl]oleylamide ($C_{17}H_{33}CO$-NH-$C_2H_4$-NH-$C_2H_4$-$NH_2$) as a main ingredient. The composition of the Reaction Product A13 was analyzed by means of liquid chromatography (LC). As a result, a proportion of N-[2-[(2-aminoethyl)amino]ethyl]oleylamide to bis(2-(oleylamido)ethyl)amine was found to be 61/39 (mass ratio).

The liquid chromatography was carried out under the same measurement conditions as in Production Example A11.

Comparative Production Example A1 [Synthesis of N-[2-[(2-Aminoethyl)amino]ethyl]octylamide]

The reaction was performed in the same manner as in Production Example A1, except for using 28.8 g (0.2 moles) of octanoic acid in place of 34.5 g (0.2 moles) of the decanoic acid, thereby obtaining Comparative Reaction Product A1 containing N-[2-[(2-aminoethyl)amino]ethyl]octylamide ($C_7H_{15}CO$-NH-$C_2H_4$-NH-$C_2H_4$-$NH_2$) as a main ingredient.

Comparative Production Example A2 [Synthesis of N-[2-[(2-Aminoethyl)amino]ethyl](2-heptylundecyl)amide]

The reaction was performed in the same manner as in Production Example A4, except for using 56.9 g (0.2 moles) of 2-heptylundecanoic acid in place of 51.3 g (0.2 moles) of the hexadecanoic acid, thereby obtaining Comparative Reaction Product A2 containing N-[2-[(2-aminoethyl)amino]ethyl](2-heptylundecyl)amide ((2-$C_7H_{15}$)-$C_{10}H_{20}CO$-NH-$C_2H_4$-NH-$C_2H_4$-$NH_2$) as a main ingredient.

Comparative Production Example A3 [Synthesis of N-[2-[(2-Aminoethyl)amino]ethyl](2,2,4,8,10,10-hexamethylundecyl)amide]

The reaction was performed in the same manner as in Production Example A4, except for using 56.9 g (0.2 moles) of 2,2,4,8,10,10-hexamethylundecanoic acid in place of 51.3 g (0.2 moles) of the hexadecanoic acid, thereby obtaining Comparative Reaction Product A3 containing N-[2-[(2-aminoethyl)amino]ethyl](2,2,4,8,10,10-hexamethylundecyl)amide ((2,2,4,8,10,10-$(CH_3)_6$-$C_{10}H_{15}CO$-NH-$C_2H_4$-NH-$C_2H_4$-$NH_2$) as a main ingredient.

Comparative Production Example A4 [Synthesis of Bis(2-decylamido)ethyl]amine]

The reaction was performed in the same manner as in Production Example A1, except for using 10.3 g (0.1 moles) of diethylenetriamine in place of 103.2 g (1.0 mol) of the diethylenetriamine, thereby obtaining Comparative Reaction Product A4 containing bis(2-decylamido)ethyl]amine ($C_9H_{19}CO$-NH-$C_2H_4$-NH-$C_2H_4$-NH-CO-$C_9H_{19}$) as a main ingredient.

Comparative Production Example A5 [Synthesis of Bis(2-isostearylamido)ethyl]amine]

The reaction was performed in the same manner as in Production Example A1, except for using 10.3 g (0.1 moles) of diethylenetriamine in place of 103.2 g (1.0 mol) of the diethylenetriamine and 56.9 g (0.2 moles) of isostearic acid in place of 34.5 g (0.2 moles) of the decanoic acid, respectively, thereby obtaining Comparative Reaction Product A5 containing bis(2-isostearylamido)ethyl]amine (iso-$C_{17}H_{35}CO$-NH-$C_2H_4$-NH-$C_2H_4$-NH-CO-iso-$C_{17}H_{35}$) as a main ingredient.

Comparative Production Example A6 [Synthesis of Poly(isostearyl)amide]

The reaction was performed in the same manner as in Production Example A1, except for using 18.9 g (0.1 moles) of tetraethylenepentamine in place of 103.2 g (1.0 mol) of the diethylenetriamine and 85.3 g (0.3 moles) of isostearic acid in place of 34.5 g (0.2 moles) of the decanoic acid, respectively, thereby obtaining Comparative Reaction Product A6 containing a polyamide composed of isostearic acid and tetraethylenepentamine as a main ingredient.

Comparative Production Example A7 [Synthesis of Aminoethyldecylamide]

The reaction was performed in the same manner as in Production Example A1, except for using 60.1 g (1.0 mol) of ethylenediamine in place of 103.2 g (1.0 mol) of the diethylenetriamine, thereby obtaining Comparative Reaction Product A7 containing aminoethyldecylamide ($C_9H_{19}CO-NH-C_2H_4-NH_2$) as a main ingredient.

The charged amounts of the amines and the fatty acids and the ratios thereof in Production Examples A1 to A13 and Comparative Production Examples A1 to A7 are shown in Tables 1 and 2.

Table 1

| | | Production Example | | | | | | | | | | | | |
|---|---|---|---|---|---|---|---|---|---|---|---|---|---|---|
| | | A1 | A2 | A3 | A4 | A5 | A6 | A7 | A8 | A9 | A10 | A11 | A12 | A13 |
| Charged amount of amine (mol) | Diethylenetriamine | 1.0 | 1.0 | 1.0 | 0.8 | 0.8 | 0.8 | 0.8 | | | | 0.6 | 0.4 | 0.2 |
| | Triethylenetetramine | | | | | | | | 1.0 | 0.8 | | | | |
| | Tetraethylenepentamine | Tetraethylenepentamine - | | | | | | | | | 0.8 | | | |
| Charged amount of fatty acid (mol) | Decanoic acid | 0.2 | | | | | | | 0.2 | | | | | |
| | Dodecanoic acid | | 0.2 | | | | | | | | | | | |
| | Tetradecanoic acid | | | 0.2 | | | | | | | | | | |
| | Hexadecanoic acid | | | | 0.2 | | | | | | | | | |
| | Stearic acid | | | | | 0.2 | | | | | | | | |
| | Oleic acid | | | | | | 0.2 | | | 0.2 | 0.2 | 0.2 | 0.2 | 0.2 |
| | Erucic acid | | | | | | | 0.2 | | | | | | |
| Amine/fatty acid molar ratio | | 5.0 | 5.0 | 5.0 | 4.0 | 4.0 | 4.0 | 4.0 | 5.0 | 4.0 | 4.0 | 3.0 | 2.0 | 1.0 |

Table 2

| | | Comparative Production Example | | | | | | |
|---|---|---|---|---|---|---|---|---|
| | | A1 | A2 | A3 | A4 | A5 | A6 | A7 |
| Charged amount of amine (mol) | Diethylenetriamine | 1.0 | 0.8 | 0.8 | 0.1 | 0.1 | | |
| | Tetraethylenepentamine | | | | | | 0.1 | |
| | Ethylenediamine | | | | | | | 1.0 |
| Charged amount of fatty acid (mol) | Decanoic acid | | | | 0.2 | | | 0.2 |
| | Octanoic acid | 0.2 | | | | | | |
| | 2-Heptylundecanoic acid | | 0.2 | | | | | |
| | Hexamethylundecanoic acid | | | 0.2 | | | | |
| | Isostearic acid | | | | | 0.2 | 0.3 | |
| Amine/fatty acid molar ratio | | 5.0 | 4.0 1 | 4.0 | 0.5 | 0.5 | 0.3 | 5.0 |

Examples A1 to A13 and Comparative Examples A1 to A13

Lubricating oil compositions having blending compositions shown in Tables 3 and 4 were prepared and evaluated with respect to an intermetal friction coefficient and intermetal friction-coefficient/slipping-velocity characteristics by the following evaluation methods. The results are shown in Tables 3 and 4.

The aminoethyldecylamide synthesized in Comparative Production Example A7 and commercially available dodecylamide could not be evaluated because they were insoluble in the lubricant base oil.

[Evaluation Methods]

(1) Intermetal Friction Coefficient

The intermetal friction coefficient was evaluated using a reciprocating cylinder-on-block tribometer (manufactured by Cameron-Plint). As the intermetal friction coefficient is higher, the transmission torque capacity becomes larger. The evaluation conditions are as follows.

<Running-in Conditions>

Surface pressure: 0.8 GPa
Oil temperature: 100°C
Average slipping velocity: 0.16 m/s
Amplitude: 8 mm
Time: 10 minutes

<Measurement Conditions>

Surface pressure: 0.8 GPa
Oil temperature: 100°C
Average slipping velocity: 0.16 m/s
Amplitude: 8 mm

<Test Piece Material>

Steel (block)-steel (cylinder: diameter = 15 mm, contact length = 5 mm)

(2) Intermetal Friction-Coefficient/Slipping-Velocity Characteristics

The intermetal friction-coefficient/slipping-velocity characteristics were evaluated using a reciprocating cylinder-on-block tribometer (manufactured by Cameron-Plint). As the value of the intermetal friction-coefficient/slipping-velocity characteristics is larger, the vibration or noise is hardly generated. The evaluation conditions are as follows.

<Running-in Conditions>

Surface pressure: 0.8 GPa
Oil temperature: 100°C
Average slipping velocity: 0.16 m/s
Amplitude: 8 mm
Time: 10 minutes

<Measurement Conditions>

Surface pressure: 0.8 GPa
Oil temperature: 100°C
Average slipping velocity: 0.032 m/s and 0.064 m/s
Amplitude: 8 mm

<Test Piece Material>

Steel (block)-steel (cylinder: diameter = 15 mm, contact length = 5 mm)

<Calculation Method of Intermetal Friction-Coefficient/Slipping-Velocity Characteristics>

Intermetal friction-coefficient/slipping-velocity characteristics = {(Friction coefficient at 0.064 m/s) - (Friction coefficient at 0.032 m/s)}/(0.064 m/s - 0.032 m/s)

Table 3

| | | Example | | | | | | | | | | | | |
|---|---|---|---|---|---|---|---|---|---|---|---|---|---|---|
| | | A1 | A2 | A3 | A4 | A5 | A6 | A7 | A8 | A9 | A10 | A11 | A12 | A13 |
| Blending composition (mass%) | Base oil | 78.1 | 78.1 | 78.1 | 78.1 | 78.1 | 78.1 | 78.1 | 78.1 | 78.1 | 78.1 | 78.1 | 78.1 | 78.1 |
| | Reaction Product 1 | 0.5 | | | | | | | | | | | | |
| | Reaction Product 2 | | 0.5 | | | | | | | | | | | |
| | Reaction Product 3 | | | 0.5 | | | | | | | | | | |
| | Reaction Product 4 | | | | 0.5 | | | | | | | | | |
| | Reaction Product 5 | | | | | 0.5 | | | | | | | | |
| | Reaction Product 6 | | | | | | 0.5 | | | | | | | |
| | Reaction Product 7 | | | | | | | 0.5 | | | | | | |
| | Reaction Product 8 | | | | | | | | 0.5 | | | | | |
| | Reaction Product 9 | | | | | | | | | 0.5 | | | | |
| | Reaction Product 10 | | | | | | | | | | 0.5 | | | |
| | Reaction Product 11 | | | | | | | | | | | 0.5 | | |
| | Reaction Product 12 | | | | | | | | | | | | 0.5 | |
| | Reaction Product 13 | | | | | | | | | | | | | 0.5 |
| | Additive package | 21.4 | 21.4 | 21.4 | 21.4 | 21.4 | 21.4 | 21.4 | 21.4 | 21.4 | 21.4 | 21.4 | 21.4 | 21.4 |
| Evaluation results | Intermetal friction coefficient | 0.110 | 0.106 | 0.103 | 0.101 | 0.096 · | 0.106 | 0.099 | 0.113 | 0.105 | 0.103 | 0.106 | 0.105 | 0.105 |
| | Intermetal friction-coefficient/ slipping-velocity characteristics | 0.096 | 0.102 | 0.111 | 0.116 | 0.125 | 0.110 | 0.119 | 0.074 | 0.098 | 0.071 | 0.111 | 0.107 | 0.101 |

Table 4

| | | Comparative Example | | | | | | | | | | | | |
|---|---|---|---|---|---|---|---|---|---|---|---|---|---|---|
| | | A1 | A2 | A3 | A4 | A5 | A6 | A7 | A8 | A9 | A10 | A11 | A12 | A13 |
| Blending composition (mass%) | Base oil | 78.1 | 78.1 | 78.1 | 78.1 | 78.1 | 78.1 | 78.1 | 78.1 | 78.1 | 78.1 | 78.1 | 78.1 | 78.6 |
| | Comparative Reaction Product 1 | 0.5 | | | | | | | | | | | | |
| | Comparative Reaction Product 2 | | 0.5 | | | | | | | | | | | |
| | Comparative Reaction Product 3 | | | 0.5 | | | | | | | | | | |
| | Comparative Reaction Product 4 | | | | 0.5 | | | | | | | | | |
| | Comparative Reaction Product 5 | | | | | 0.5 | | | | | | | | |
| | Comparative Reaction Product 6 | | | | | | 0.5 | | | | | | | |
| | Comparative Reaction Product 7 | | | | | | | 0.5 | | | | | | |
| | Commercially available polyamide | | | | | | | | 0.5 | | | | | |
| | Commercially available fatty acid | | | | | | | | | 0.5 | | | | |
| | Commercially available amine | | | | | | | | | | 0.5 | | | |
| | Commercially available alcohol | | | | | | | | | | | 0.5 | | |
| | Commercially available amide | | | | | | | | | | | | 0.5 | |
| | Additive package | 21.4 | 21.4 | 21.4 | 21.4 | 21.4 | 21.4 | 21.4 | 21.4 | 21.4 | 21.4 | 21.4 | 21.4 | 21.4 |

(continued)

| | | Comparative Example | | | | | | | | | | | | |
|---|---|---|---|---|---|---|---|---|---|---|---|---|---|---|
| | | A1 | A2 | A3 | A4 | A5 | A6 | A7 | A8 | A9 | A10 | A11 | A12 | A13 |
| Evaluation results | Intermetal friction coefficient | 0.116 | 0.127 | 0.134 | 0.113 | 0.110 | 0.112 | Insoluble | 0.118 | 0.107 | 0.080 | 0.113 | Insoluble | 0.132 |
| | Intermetal friction-coefficient/ slipping-velocity characteristics | 0.060 | 0.031 | 0.030 | 0.065 | 0.053 | 0.045 | | 0.050 | 0.024 | 0.100 | 0.037 | | 0.001 |

Details of the base oil and additives used in the aforementioned Examples A1 to A13 and Comparative Examples A1 to A13 are as follows.

Base oil: Paraffinic mineral oil (kinematic viscosity at 40°C: 21 $mm^2/s$, kinematic viscosity at 100°C: 4.5 $mm^2/s$, viscosity index: 130)
Commercially available polyamide: Fatty acid polyamide
Commercially available fatty acid: Oleic acid
Commercially available amine: Decylamine
Commercially available alcohol: Glycerol monooleate
Commercially available amide: Dodecylamide
Additive package: Additive package containing metal-based detergent, ashless dispersant, phosphorus-based extreme pressure agent, sulfur-based extreme pressure agent, and antioxidant

[Evaluation Results]

As shown in Tables 3 and 4, the lubricating oil compositions, in which the lubricating oil additive of the present invention was blended (Examples A1 to A13), are high in the intermetal friction coefficient and excellent in the intermetal friction-coefficient/slipping-velocity characteristics.

On the other hand, the lubricating oil compositions of Comparative Examples A1 to A6, A8 to A11, and A13 were not sufficient in either one or both of characteristics of the intermetal friction coefficient and the intermetal friction-coefficient/slipping-velocity characteristics. For example, in Comparative Example A13, the lubricating oil composition, in which a friction modifier was not blended, was quite inferior in the intermetal friction-coefficient/slipping-velocity characteristics. In Comparative Example A1, though the additive having an alky group having 7 carbon atoms was blended, the intermetal friction-coefficient/slipping-velocity characteristics could not be satisfied. In Comparative Examples A2 and A3, in which the additive having a largely branched alkyl group was blended, though the intermetal friction coefficient was good, an effect for improving the intermetal friction-coefficient/slipping-velocity characteristics was not observed. In Comparative Examples A4, A5, and A6, in which the additive having been made free from a terminal amine structure through condensation of plural fatty acids was blended, an effect for improving the intermetal friction-coefficient/slipping-velocity characteristics was not observed. Comparative Examples A8 to A11, in which the commercially available friction modifier was blended, were not sufficient in either one or both of characteristics of the intermetal friction coefficient and the intermetal friction-coefficient/slipping-velocity characteristics.

<Examples Regarding Second Lubricating Oil Additive>

Production Example B1 [Synthesis of N-[2-[(2-Aminoethyl)amino]ethyl]isostearylamide]

In a 200-mL four-neck round bottom flask, 82.6 g (0.8 moles) of diethylenetriamine was charged and subjected to temperature rise to about 90°C in a nitrogen gas stream. Subsequently, 56.9 g (0.2 moles) of isostearic acid of a methyl-branched type was gradually added dropwise in the flask. After stirring for 30 minutes such that the system became uniform, the resultant was subjected to temperature rise to 200°C and then subjected to reaction for 2 hours. Furthermore, the contents of the flask were subjected to temperature drop to 150°C, and the excessive diethylenetriamine and residual water were distilled off while gradually evacuating the inside of the flask, thereby obtaining Reaction Product B1 containing N-[2-[(2-aminoethyl)amino]ethyl]isostearylamide (iso-$C_{17}H_{35}CO-NH-C_2H_4-NH-C_2H_4-NH_2$) as a main ingredient. The presence of the amide compound was confirmed by subjecting the Reaction Product B1 to $^1H$-NMR. $^1H$-NMR spectra as measured with respect to the Reaction Product B1 are shown below.

$^1H$-NMR Spectra ($CDCl_3$, 0.03% TMS)

| δ Value (ppm) | Splitting | Assignment | Integration ratio (theoretical ratio) |
|---|---|---|---|
| 0.8 to 0.9 | m | H of methyl of isostearyl group | 7.2 (33) |
| 1.2 to 1.4 | s | H of methylene of isostearyl group | 25.8 |
| | | H of terminal amino group of diethylenetriamine | |
| 1.6 to 1.7 | bs | H of β-position methylene of isostearyl group | 3.1 (3) |
| | | H of intermediate amino group of diethylenetriamine | |
| 2.1 to 2.3 | m | H of α-position methylene of isostearyl group | 2.2 (2) |
| 3.1 to 3.4 | m | H of methylene of diethylenetriamine | 5.6 (6) |

(continued)

| δ Value (ppm) | Splitting | Assignment | Integration ratio (theoretical ratio) |
|---|---|---|---|
| 3.7 to 3.8 | m | H of amide-side methylene of diethylenetriamine | 1.7 (2) |
| 5.6 to 5.7 | s | H of amide group | 0.7 (1) |

Production Example B2 [Synthesis of N-[2-[[2-[(2-Aminoethyl)amino]ethyl]amino]ethyl]isostearylamide]

The reaction was performed in the same manner as in Production Example B1, except for using 117.0 g (0.8 moles) of triethylenetetramine in place of 82.6 g (0.8 moles) of the diethylenetriamine, thereby obtaining Reaction Product B2 containing N-[2-[[2-[(2-aminoethyl)amino]ethyl]amino]ethyl]isostearylamide (iso-$C_{17}H_{35}CO-NH-C_2H_4-NH-C_2H_4-NH-C_2H_4-NH_2$) as a main ingredient. The presence of the amide compound was confirmed by means of $^1$H-NMR.

Comparative Production Example B1 [Synthesis of N-[2[(2-Aminoethyl)amino]ethyl](2-heptylundecyl)amide]

The reaction was performed in the same manner as in Production Example B1, except for using 56.9 g (0.2 moles) of 2-heptylundecanoic acid in place of 56.9 g (0.2 moles) of the isostearic acid of a methyl-branched type, thereby obtaining Comparative Reaction Product B1 containing N-[2-[(2-aminoethyl)amino]ethyl](2-heptylundecyl)amide ((2-$C_7H_{15}$)-$C_{10}H_{20}CO-NH-C_2H_4-NH-C_2H_4-NH_2$) as a main ingredient.

Comparative Production Example B2 [Synthesis of N-[2-[(2-Aminoethyl)amino]ethyl](2,2,4,8,10,10-hexamethylundecyl)amide]

The reaction was performed in the same manner as in Production Example B1, except for using 56.9 g (0.2 moles) of 2,2,4,8,10,10-hexamethylundecanoic acid in place of 56.9 g (0.2 moles) of the isostearic acid of a methyl-branched type, thereby obtaining Comparative Reaction Product B2 containing N-[2-[(2-aminoethyl)amino]ethyl](2,2,4,8,10,10-hexamethylundecyl)amide (2,2,4,8,10,10-$(CH_3)_6-C_{10}H_{15}CO-NH-C_2H_4-NH-C_2H_4-NH_2$) as a main ingredient.

Comparative Production Example B3 [Synthesis of Bis(2-(isostearylamido)ethyl)amine]

The reaction was performed in the same manner as in Production Example B1, except for using 10.3 g (0.1 moles) of diethylenetriamine in place of 82.6 g (0.8 moles) of the diethylenetriamine, thereby obtaining Comparative Reaction Product B3 containing bis(2-(isostearylamido)ethyl)amine (iso-$C_{17}H_{35}CO-NH-C_2H_4-NH-C_2H_4-NH-CO$-iso-$C_{17}H_{35}$) as a main ingredient.

Comparative Production Example B4 [Synthesis of Poly(isostearyl)amide]

The reaction was performed in the same manner as in Production Example B1, except for using 18.9 g (0.1 moles) of tetraethylenepentamine in place of 103.2 g (1.0 mol) of the diethylenetriamine and 85.3 g (0.3 moles) of isostearic acid in place of 56.9 g (0.2 moles) of the isostearic acid, respectively, thereby obtaining Comparative Reaction Product B4 containing a polyamide composed of isostearic acid and tetraethylenepentamine as a main ingredient.

The charged amounts of the amines and the fatty acids and the ratios thereof in Production Examples B1 to B2 and Comparative Production Examples B1 to B4 are shown in Table 5.

Table 5

| | | Production Example | | Comparative Production Example | | | |
|---|---|---|---|---|---|---|---|
| | | B1 | B2 | B1 | B2 | B3 | B4 |
| Charged amount of amine (mol) | Diethylenetriamine | 0.8 | | 0.8 | 0.8 | 0.1 | |
| | Triethylenetetramine | | 0.8 | | | | |
| | Tetraethylenepentamine | | | | | | 0.1 |

(continued)

| | | Production Example | | Comparative Production Example | | | |
|---|---|---|---|---|---|---|---|
| | | B1 | B2 | B1 | B2 | B3 | B4 |
| Charged amount of fatty acid (mol) | Isostearic acid of methyl-branched type | 0.2 | 0.2 | | | 0.2 | 0.3 |
| | 2-Heptylundecanoic acid | | | 0.2 | | | |
| | Hexamethylundecanoic acid | | | | 0.2 | | |
| Amine/fatty acid molar ratio | | 4.0 | 4.0 | 4.0 | 4.0 | 0.5 | 0.3 |

Examples B1 to B2 and Comparative Examples B1 to B9

Lubricating oil compositions having blending compositions shown in Table 6 were prepared and evaluated with respect to an intermetal friction coefficient and intermetal friction-coefficient/slipping-velocity characteristics by the following evaluation methods. The results are shown in Table 6.

[Evaluation Methods]

(1) Intermetal Friction Coefficient

The intermetal friction coefficient was evaluated using a reciprocating cylinder-on-block tribometer (manufactured by Cameron-Plint). As the intermetal friction coefficient is higher, the transmission torque capacity becomes larger. The evaluation conditions are as follows.

<Running-in Conditions>

Surface pressure: 0.8 GPa
Oil temperature: 100°C
Average slipping velocity: 0.16 m/s
Amplitude: 8 mm
Time: 10 minutes

<Measurement Conditions>

Surface pressure: 0.8 GPa
Oil temperature: 100°C
Average slipping velocity: 0.16 m/s
Amplitude: 8 mm

<Test Piece Material>

Steel (block)-steel (cylinder: diameter = 15 mm, contact length = 5 mm)

(2) Intermetal Friction-Coefficient/Slipping-Velocity Characteristics

The intermetal friction-coefficient/slipping-velocity characteristics were evaluated using a reciprocating cylinder-on-block tribometer (manufactured by Cameron-Plint). As the value of the intermetal friction-coefficient/slipping-velocity characteristics is larger, the vibration or noise is hardly generated. The evaluation conditions are as follows.

<Running-in Conditions>

Surface pressure: 0.8 GPa
Oil temperature: 100°C
Average slipping velocity: 0.16 m/s
Amplitude: 8 mm
Time: 10 minutes

<Measurement Conditions>

Surface pressure: 0.8 GPa
Oil temperature: 100°C
Average slipping velocity: 0.032 m/s and 0.064 m/s
Amplitude: 8 mm

<Test Piece Material>

Steel (block)-steel (cylinder: diameter = 15 mm, contact length = 5 mm)

<Calculation Method of Intermetal Friction-Coefficient/Slipping-Velocity Characteristics>

Intermetal friction-coefficient/slipping-velocity characteristics = {(Friction coefficient at 0.064 m/s) - (Friction coefficient at 0.032 m/s)}/(0.064 m/s - 0.032 m/s)

Table 6

| | | Example | | Com parative Example | | | | | | | | |
|---|---|---|---|---|---|---|---|---|---|---|---|---|
| | | B1 | B2 | B1 | B2 | B3 | B4 | B5 | B6 | B7 | B8 | B9 |
| Blending composition (mass%) | Base oil | 78.1 | 78.1 | 78.1 | 78.1 | 78.1 | 78.1 | 78.1 | 78.1 | 78.1 | 78.1 | 78.6 |
| | Reaction Product 1 | 0.5 | | | | | | | | | | |
| | Reaction Product 2 | | 0.5 | | | | | | | | | |
| | Comparative Reaction Product 1 | | | 0.5 | | | | | | | | |
| | Comparative Reaction Product 2 | | | | 0.5 | | | | | | | |
| | Comparative Reaction Product 3 | | | | | 0.5 | | | | | | |
| | Comparative Reaction Product 4 | | | | | | 0.5 | | | | | |
| | Commercially available polyamide | | | | | | | 0.5 | | | | |
| | Commercially available fatty acid | | | | | | | | 0.5 | | | |
| | Commercially available amine | | | | | | | | | 0.5 | | |
| | Commercially available alcohol | | | | | | | | | | 0.5 | |
| | Additive package | 21.4 | 21.4 | 21.4 | 21.4 | 21.4 | 21.4 | 21.4 | 21.4 | 21.4 | 21.4 | 21.4 |
| Evaluation results | Intermetal friction coefficient | 0.110 | 0.110 | 0.127 | 0.134 | 0.110 | 0.112 | 0.118 | 0.107 | 0.080 | 0.113 | 0.132 |
| | Intermetal friction-coefficient/ slipping-velocity characteristics | 0.075 | 0.070 | 0.031 | 0.030 | 0.053 | 0.045 | 0.050 | 0.024 | 0.100 | 0.037 | 0.001 |

Details of the base oil and additives used in the aforementioned Examples B1 to B2 and Comparative Examples B1 to B9 are as follows.

Base oil: Paraffinic mineral oil (kinematic viscosity at 40°C: 21 $mm^2/s$, kinematic viscosity at 100°C: 4.5 $mm^2/s$, viscosity index: 130)
Commercially available polyamide: Fatty acid polyamide
Commercially available fatty acid: Oleic acid
Commercially available amine: Decylamine
Commercially available alcohol: Glycerol monooleate
Additive package: Additive package containing metal-based detergent, ashless dispersant, phosphorus-based extreme pressure agent, sulfur-based extreme pressure agent, and antioxidant

[Evaluation Results]

As shown in Table 6, the lubricating oil compositions, in which the lubricating oil additive of the present invention was blended (Examples B1 to B2), are high in the intermetal friction coefficient and excellent in the intermetal friction-coefficient/slipping-velocity characteristics.

On the other hand, the lubricating oil compositions of Comparative Examples B1 to B9 were not sufficient in either one or both of characteristics of the intermetal friction coefficient and the intermetal friction-coefficient/slipping-velocity characteristics. For example, in Comparative Example B9, the lubricating oil composition, in which a friction modifier was not blended, was quite inferior in the intermetal friction-coefficient/slipping-velocity characteristics. In Comparative Examples B1 and B2, in which the additive having a largely branched or multi-branched alkyl group was blended, though the intermetal friction coefficient was good, an effect for improving the intermetal friction-coefficient/slipping-velocity characteristics was not observed. In Comparative Examples B3 and B4, in which the additive having been made free from a terminal amine structure through condensation of plural fatty acids was blended, an effect for improving the intermetal friction-coefficient/slipping-velocity characteristics was not observed. Comparative Examples B5 to B8, in which the commercially available friction modifier was blended, were not sufficient in either one or both of characteristics of the intermetal friction coefficient and the intermetal friction-coefficient/slipping-velocity characteristics.

<Examples Regarding Third Lubricating Oil Additive>

Production Example C1 [Synthesis of 2-(1-Piperazinyl)ethyldecylamide]

In a 200-mL four-neck round bottom flask, 77.5 g (0.6 moles) of aminoethylpiperazine was charged and subjected to temperature rise to about 90°C in a nitrogen gas stream. Subsequently, 34.5 g (0.2 moles) of decanoic acid was gradually added dropwise in the flask. After stirring for 30 minutes such that the system became uniform, the resultant was subjected to temperature rise to 200°C and then subjected to reaction for 2 hours. Furthermore, the contents of the flask were subjected to temperature drop to 150°C, and the excessive aminoethylpiperazine and residual water were distilled off while gradually evacuating the inside of the flask, thereby obtaining Reaction Product C1 containing 2-(1-piperazinyl)ethyldecylamide described below as a main ingredient. The presence of the amide compound was confirmed by subjecting the Reaction Product C1 to $^1H$-NMR. $^1H$-NMR spectra as measured with respect to the Reaction Product C1 are shown below.

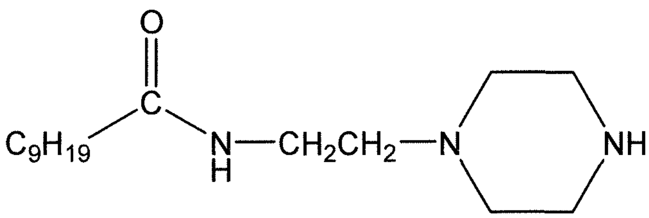

$^1H$-NMR Spectra ($CDCl_3$, 0.03% TMS)

| δ Value (ppm) | Splitting | Assignment | Integration ratio (theoretical ratio) |
|---|---|---|---|
| 0.8 to 0.9 | t | H of methyl of decyl group | 3.2 (3) |
| 1.2 to 1.4 | s | H of methylene of decyl group<br>H of terminal amino group of piperazine | 14.1 (13) |

(continued)

| δ Value (ppm) | Splitting | Assignment | Integration ratio (theoretical ratio) |
|---|---|---|---|
| 1.5 to 1.7 | bs | H of β-position methylene of decyl group | 3.2 (2) |
| 2.1 to 2.3 | m | H of α-position methylene of decyl group | 2.0 (2) |
| 2.5 to 2.6 | m | H of methylene of piperazine | 5.7 (8) |
| 2.9 to 3.0 | m | H of amide-side methylene of ethyleneamine | 1.8 (2) |
| 3.3 to 3.4 | m | H of methylene of ethyleneamine | 1.8 (2) |
| 5.9 to 6.0 | s | H of amide group | 0.8 (1) |

Production Example C2 [Synthesis of 2-(1-Piperazinyl)ethyldodecylamide]

The reaction was performed in the same manner as in Production Example C1, except for using 40.1 g (0.2 moles) of dodecanoic acid in place of 34.5 g (0.2 moles) of the decanoic acid, thereby obtaining Reaction Product C2 containing 2-(1-piperazinyl)ethyldodecylamide described below as a main ingredient. The presence of the amide compound was confirmed by means of $^1$H-NMR.

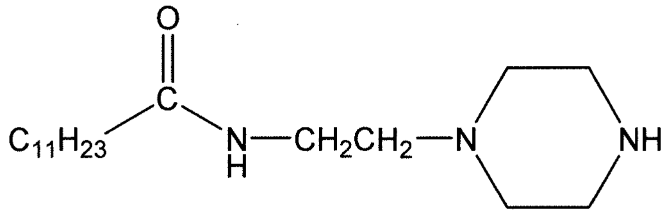

Production Example C3 [Synthesis of 2-(1-Piperazinyl)ethyloleylamide]

The reaction was performed in the same manner as in Production Example C1, except for using 56.5 g (0.2 moles) of oleic acid in place of 34.5 g (0.2 moles) of the decanoic acid, thereby obtaining Reaction Product C3 containing 2-(1-piperazinyl)ethyloleylamide described below as a main ingredient. The presence of the amide compound was confirmed by means of $^1$H-NMR.

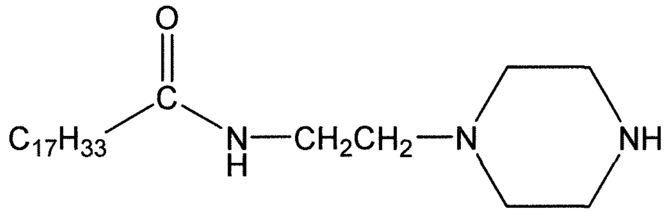

Production Example C4 [Synthesis of 2-(1-Piperazinyl)ethylisostearylamide]

The reaction was performed in the same manner as in Production Example C1, except for using 56.9 g (0.2 moles) of isostearic acid of a methyl-branched type in place of 34.5 g (0.2 moles) of the decanoic acid, thereby obtaining Reaction Product C4 containing 2-(1-piperazinyl)ethylisostearylamide described below as a main ingredient. The presence of the amide compound was confirmed by means of $^1$H-NMR.

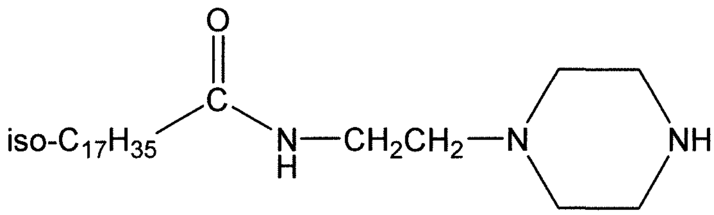

Comparative Production Example C1 [Synthesis of N-[2-[(2-Aminoethyl)amino]ethyl](2-heptylundecyl)amide]

The reaction was performed in the same manner as in Production Example C1, except for using 82.6 g (0.8 moles) of diethylenetriamine in place of 77.5 g (0.6 moles) of the aminoethylpiperazine and 56.9 g (0.2 moles) of 2-heptylundecanoic acid in place of 34.5 g (0.2 moles) of the decanoic acid, respectively, thereby obtaining Comparative Reaction Product C1 containing N-[2-[(2-aminoethyl)amino]ethyl](2-heptylundecyl)amide ((2-$C_7H_{15}$)-$C_{10}H_{20}$CO-NH-$C_2H_4$-NH-$C_2H_4$-$NH_2$) as a main ingredient.

Comparative Production Example C2 [Synthesis of N-[2-[(2-Aminoethyl)amino]ethyl](2,2,4,8,10,10-hexamethylundecyl)amide]

The reaction was performed in the same manner as in Comparative Production Example C1, except for using 56.9 g (0.2 moles) of 2,2,4,8,10,10-hexamethylundecanoic acid in place of 56.9 g (0.2 moles) of the 2-heptylundecanoic acid, thereby obtaining Comparative Reaction Product C2 containing N-[2-[(2-aminoethyl)amino]ethyl](2,2,4,8,10,10-hexamethylundecyl)amide ((2,2,4,8,10,10-$(CH_3)_6$-$C_{10}H_{15}$CO-NH-$C_2H_4$-NH-$C_2H_4$-$NH_2$) as a main ingredient.

Comparative Production Example C3 [Synthesis of 2-(1-Piperazinyl)ethyl(2-heptylundecyl)amide]

The reaction was performed in the same manner as in Production Example C1, except for using 56.9 g (0.2 moles) of 2-heptylundecanoic acid in place of 34.5 g (0.2 moles) of the decanoic acid, thereby obtaining Comparative Reaction Product C3 containing 2-(1-piperazinyl)ethyl(2-heptylundecyl)amide as a main ingredient.

Comparative Production Example C4 [Synthesis of Bis(2-(isostearylamido)ethyl)amine]

The reaction was performed in the same manner as in Production Example C1, except for using 10.3 g (0.1 moles) of diethylenetriamine in place of 77.5 g (0.6 moles) of the aminoethylpiperazine and 56.9 g (0.2 moles) of the isostearic acid in place of 34.5 g (0.2 moles) of the decanoic acid, respectively, thereby obtaining Comparative Reaction Product C4 containing bis(2-(isostearylamido)ethyl)amine (iso-$C_{17}H_{35}$CO-NH-$C_2H_4$-NH-$C_2H_4$-NH-CO-iso-$C_{17}H_{35}$) as a main ingredient.

Comparative Production Example C5 [Synthesis of Poly(isostearyl)amide]

The reaction was performed in the same manner as in Production Example C1, except for using 18.9 g (0.1 moles) of tetraethylenepentamine in place of 77.5 g (0.6 moles) of the aminoethylpiperazine and 85.3 g (0.3 moles) of isostearic acid in place of 34.5 g (0.2 moles) of the decanoic acid, respectively, thereby obtaining Comparative Reaction Product C5 containing a polyamide composed of isostearic acid and tetraethylenepentamine as a main ingredient.

Comparative Production Example C6 [Synthesis of (5-Amino-3-heptadecenyl-1,2,4-triazole)]

In a 300-mL flask, 6.08 g (0.05 moles) of aminoguanidine hydrochloride, 20 mL of water, 80 mL of 1,4-dioxane, 4.62 g (0.116 moles) of sodium hydroxide were charged and stirred in a nitrogen gas stream for 0.5 hours. Subsequently, 15.04 g (0.05 moles) of oleyl chloride having been diluted with 20 mL of 1,4-dioxane was gradually added dropwise such that the temperature did not exceed 35°C. The contents were reacted with each other at 20 to 35°C for 2 hours, and the temperature was then raised to 130°C while removing the water and 1,4-dioxane, thereby performing the reaction for 4 hours. 200 mL of toluene was added, stirring was performed at 100°C to dissolve a reaction product, and a salt was removed by means of suction filtration. The toluene was distilled off to obtain the target material (5-amino-3-heptadecenyl-1,2,4-triazole).

The charged amounts of the amines and the fatty acids and the ratios thereof in Production Examples C1 to C4 and Comparative Production Examples C1 to C5 are shown in Table 7.

Table 7

| | | Production Example | | | | Comparative Production Example | | | | |
|---|---|---|---|---|---|---|---|---|---|---|
| | | C1 | C2 | C3 | C4 | C1 | C2 | C3 | C4 | C5 |
| Charged amount of amine (mol) | Aminoethylpiperazine | 0.6 | 0.6 | 0.6 | 0.6 | | | 0.6 | | |
| | Diethylenetriamine | | | | | 0.8 | 0.8 | | 0.1 | |
| | Tetraethylenepentamine | | | | | | | | | 0.1 |
| Charged amount of fatty acid (mol) | Decanoic acid | 0.2 | | | | | | | | |
| | Dodecanoic acid | | 0.2 | | | | | | | |
| | Oleic acid | | | 0.2 | | | | | | |
| | Isostearic acid of methyl-branched type | | | | 0.2 | | | | 0.2 | 0.3 |
| | 2-Heptylundecanoic acid | | | | | 0.2 | | 0.2 | | |
| | Hexamethylundecanoic acid | | | | | | 0.2 | | | |
| Amine/fatty acid molar ratio | | 3.0 | 3.0 | 3.0 | 3.0 | 4.0 | 4.0 | 3.0 | 0.5 | 0.3 |

Examples C1 to C4 and Comparative Examples C1 to C11

Lubricating oil compositions having blending compositions shown in Tables 8 and 9 were prepared and evaluated with respect to an intermetal friction coefficient and intermetal friction-coefficient/slipping-velocity characteristics by the following evaluation methods. The results are shown in Tables 8 and 9.

[Evaluation Methods]

(1) Intermetal Friction Coefficient

The intermetal friction coefficient was evaluated using a reciprocating cylinder-on-block tribometer (manufactured by Cameron-Plint). As the intermetal friction coefficient is higher, the transmission torque capacity becomes larger. The evaluation conditions are as follows.

<Running-in Conditions>

Surface pressure: 0.8 GPa
Oil temperature: 100°C
Average slipping velocity: 0.16 m/s
Amplitude: 8 mm
Time: 10 minutes

<Measurement Conditions>

Surface pressure: 0.8 GPa
Oil temperature: 100°C
Average slipping velocity: 0.16 m/s
Amplitude: 8 mm

<Test Piece Material>

Steel (block)-steel (cylinder: diameter = 15 mm, contact length = 5 mm)

(2) Intermetal Friction-Coefficient/Slipping-Velocity Characteristics

The intermetal friction-coefficient/slipping-velocity characteristics were evaluated using a reciprocating cylinder-on-block tribometer (manufactured by Cameron-Plint). As the value of the μ gradient is larger, the vibration or noise is hardly generated. The evaluation conditions are as follows.

<Running-in Conditions>

Surface pressure: 0.8 GPa

Oil temperature: 100°C

Average slipping velocity: 0.16 m/s

Amplitude: 8 mm

Time: 10 minutes

<Measurement Conditions>

Surface pressure: 0.8 GPa

Oil temperature: 100°C

Average slipping velocity: 0.032 m/s and 0.064 m/s

Amplitude: 8 mm

<Test Piece Material>

Steel (block)-steel (cylinder: diameter = 15 mm, contact length = 5 mm)

<Calculation Method of Intermetal Friction-Coefficient/Slipping-Velocity Characteristics>

Intermetal friction-coefficient/slipping-velocity characteristics = {(Friction coefficient at 0.064 m/s) - (Friction coefficient at 0.032 m/s)}/(0.064 m/s - 0.032 m/s)

Table 8

| | | Example | | | |
|---|---|---|---|---|---|
| | | C1 | C2 | C3 | C4 |
| Blending composition (mass%) | Base oil | 78.1 | 78.1 | 78.1 | 78.1 |
| | Reaction Product 1 | 0.5 | | | |
| | Reaction Product 2 | | 0.5 | | |
| | Reaction Product 3 | | | 0.5 | |
| | Reaction Product 4 | | | | 0.5 |
| | Additive package | 21.4 | 21.4 | 21.4 | 21.4 |
| Evaluation results | Intermetal friction coefficient | 0.110 | 0.110 | 0.108 | 0.111 |
| | Intermetal friction-coefficient/ slipping-velocity characteristics | 0.075 | 0.070 | 0.095 | 0.078 |

Table 9

| | | Comparative Example | | | | | | | | | | |
|---|---|---|---|---|---|---|---|---|---|---|---|---|
| | | C1 | C2 | C3 | C4 | C5 | C6 | C7 | C8 | C9 | C10 | C11 |
| Blending composition (mass%) | Base oil | 78.1 | 78.1 | 78.1 | 78.1 | 78.1 | 78.1 | 78.1 | 78.1 | 78.1 | 78.1 | 78.6 |
| | Comparative Reaction Product 1 | 0.5 | | | | | | | | | | |
| | Comparative Reaction Product 2 | | 0.5 | | | | | | | | | |
| | Comparative Reaction Product 3 | | | 0.5 | | | | | | | | |
| | Comparative Reaction Product 4 | | | | 0.5 | | | | | | | |
| | Comparative Reaction Product 5 | | | | | 0.5 | | | | | | |
| | 5-Amino-3-heptadecenyl-1,2,4-triazole | | | | | | 0.5 | | | | | |
| | Commercially available polyamide | | | | | | | 0.5 | | | | |
| | Commercially available fatty acid | | | | | | | | 0.5 | | | |
| | Commercially available amine | | | | | | | | | 0.5 | | |
| | Commercially available alcohol | | | | | | | | | | 0.5 | |
| | Additive package | 21.4 | 21.4 | 21.4 | 21.4 | 21.4 | 21.4 | 21.4 | 21.4 | 21.4 | 21.4 | 21.4 |
| Evaluation results | Intermetal friction coefficient | 0.127 | 0.134 | 0.131 | 0.110 | 0.112 | 0.102 | 0.118 | 0.107 | 0.080 | 0.113 | 0.132 |
| | Intermetal friction-coefficient/ slipping-velocity characteristics | 0.031 | 0.030 | 0.030 | 0.053 | 0.045 | 0.058 | 0.050 | 0.024 | 0.100 | 0.037 | 0.001 |

Details of the base oil and additives used in the aforementioned Examples C1 to C4 and Comparative Examples C1 to C11 are as follows.

Base oil: Paraffinic mineral oil (kinematic viscosity at 40°C: 21 $mm^2/s$, kinematic viscosity at 100°C: 4.5 $mm^2/s$, viscosity index: 130)
Commercially available polyamide: Fatty acid polyamide
Commercially available fatty acid: Oleic acid
Commercially available amine: Decylamine
Commercially available alcohol: Glycerol monooleate
Additive package: Additive package containing metal-based detergent, ashless dispersant, phosphorus-based extreme pressure agent, sulfur-based extreme pressure agent, and antioxidant

[Evaluation Results]

As shown in Tables 8 and 9, the lubricating oil compositions, in which the lubricating oil additive of the present invention was blended (Examples C1 to C4), are high in the intermetal friction coefficient and excellent in the intermetal friction-coefficient/slipping-velocity characteristics.

On the other hand, the lubricating oil compositions of Comparative Examples C1 to C11 were not sufficient in either one or both of characteristics of the intermetal friction coefficient and the intermetal friction-coefficient/slipping-velocity characteristics. For example, in Comparative Example C11, the lubricating oil composition, in which a friction modifier was not blended, was quite inferior in the intermetal friction-coefficient/slipping-velocity characteristics. In Comparative Examples C1 to C3, in which the additive having a largely branched or multi-branched alkyl group was blended, though the intermetal friction coefficient was good, an effect for improving the intermetal friction-coefficient/slipping-velocity characteristics was not observed. In Comparative Examples C4 and C5, in which the additive having been made free from a terminal amine structure through condensation of plural fatty acids was blended, an effect for improving the intermetal friction-coefficient/slipping-velocity characteristics was not observed. In Comparative Example C6, though the triazole having an amino group was blended, an effect for improving the intermetal friction-coefficient/slipping-velocity characteristics was not observed. Comparative Examples C7 to C10, in which the commercially available friction modifier was blended, were not sufficient in either one or both of characteristics of the intermetal friction coefficient and the intermetal friction-coefficient/slipping-velocity characteristics.

INDUSTRIAL APPLICABILITY

The lubricating oil additive of the present invention and the lubricating oil composition using the same are excellent in terms of an intermetal friction coefficient and intermetal friction-coefficient/slipping-velocity characteristics, and therefore, they are useful in lubricating oil applications for a CVT fluid and so on.

## Claims

1. A lubricating oil additive comprising an amide compound represented by the following general formula (1):

$$R^a\text{-CO-(NH-}L^1)_n\text{-NR}^bR^c \qquad (1)$$

wherein $R_a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group.

2. The lubricating oil additive according to claim 1, comprising an amide compound represented by the following general formula (2):

$$R^1\text{-CO-(NH-}L^1)_n\text{-NH}_2 \qquad (2)$$

wherein $R^1$ is a straight-chain alkyl group having 9 to 21 carbon atoms or a straight-chain alkenyl group having 9 to 21 carbon atoms; $L^1$ is an alkylene group having 1 to 6 carbon atoms; and n is an integer of 2 to 4.

3. The lubricating oil additive according to claim 2, wherein $L^1$ in the general formula (2) is an ethylene group or a

trimethylene group.

4. The lubricating oil additive according to claim 1, comprising an amide compound represented by the following general formula (3):

$$R^2\text{-CO-(NH-L}^1)_n\text{-NH}_2 \qquad (3)$$

wherein $R^2$ is a branched-chain alkyl group having 9 to 21 carbon atoms or a branched-chain alkenyl group having 9 to 21 carbon atoms; $L^1$ is an alkylene group having 1 to 6 carbon atoms; and n is an integer of 2 to 4, provided that the number of branched chains which each of the alkyl group and the alkenyl group has is 3 or less, and that the branched chain is a methyl group.

5. The lubricating oil additive according to claim 4, wherein $L^1$ in the general formula (3) is an ethylene group or a trimethylene group.

6. The lubricating oil additive according to claim 1, comprising an amide compound represented by the following general formula (4):

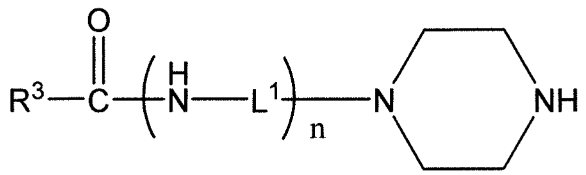
(4)

wherein $R^3$ is a straight-chain alkyl group having 8 to 22 carbon atoms, a branched-chain alkyl group having 8 to 22 carbon atoms, a straight-chain alkenyl group having 8 to 22 carbon atoms, or a branched-chain alkenyl group having 8 to 22 carbon atoms; $L^1$ is an alkylene group having 1 to 6 carbon atoms; and n is an integer of 1 to 4, provided that the number of branched chains which each of the branched-chain alkyl group and the branched-chain alkenyl group has is 3 or less, and that the branched chain is a methyl group.

7. The lubricating oil additive according to claim 6, wherein $L^1$ in the general formula (4) is an ethylene group or a trimethylene group.

8. The lubricating oil additive according to any one of claims 1 to 7, wherein the amide compound is contained in an amount of 50 mass% or more.

9. The lubricating oil additive according to any one of claims 1 to 8, which is used for a driving system oil.

10. The lubricating oil additive according to any one of claims 1 to 9, which is used for a chain type CVT or belt type CVT.

11. A lubricating oil composition comprising the lubricating oil additive according to any one of claims 1 to 10.

12. The lubricating oil composition according to claim 11, which is used for a chain type CVT or belt type CVT.

13. A method for producing the lubricating oil additive according to claim 1, comprising a step of allowing a fatty acid represented by the following general formula (5) and a polyalkylenepolyamine represented by the following general formula (6) to react with each other, the polyalkylenepolyamine being used in an amount of 1 mol or more based on 1 mol of the fatty acid:

$$R_a\text{-COOH} \qquad (5)$$

$$\text{H-(NH-L}^1)_n\text{-NR}^b\text{R}^c \qquad (6)$$

wherein $R_a$ is a straight-chain alkyl group having 8 to 22 carbon atoms or a branched-chain alkyl group having 8 to 22 carbon atoms, or a straight-chain alkenyl group having 8 to 22 carbon atoms or a branched-chain alkenyl group having 8 to 22 carbon atoms; $L^1$ is an alkylene group having 1 to 6 carbon atoms; n is an integer of 1 to 4; and $-NR^bR^c$ is a primary amino group or a piperazyl group.

**14.** The method for producing the lubricating oil additive according to claim 13, comprising a step of allowing a fatty acid represented by the following general formula (7) and a polyalkylenepolyamine represented by the following general formula (8) to react with each other, the polyalkylenepolyamine being used in an amount of 1 mol or more based on 1 mol of the fatty acid:

$R^1$-COOH (7)

H-(NH-$L^1$)$_n$-$NH_2$ (8)

wherein $R^1$ is a straight-chain alkyl group having 9 to 21 carbon atoms or a straight-chain alkenyl group having 9 to 21 carbon atom; $L^1$ is an alkylene group having 1 to 6 carbon atoms; and n is an integer of 2 to 4.

**15.** The method for producing the lubricating oil additive according to claim 13, comprising a step of allowing a fatty acid represented by the following general formula (9) and a polyalkylenepolyamine represented by the following general formula (10) to react with each other, the polyalkylenepolyamine being used in an amount of 1 mol or more based on 1 mol of the fatty acid:

$R^2$-COOH (9)

H-(NH-$L^1$)$_n$-$NH_2$ (10)

wherein $R^2$ is a branched-chain alkyl group having 9 to 21 carbon atoms or a branched-chain alkenyl group having 9 to 21 carbon atoms; $L^1$ is an alkylene group having 1 to 6 carbon atoms; and n is an integer of 2 to 4, provided that the number of branched chains which each of the alkyl group and the alkenyl group has is 3 or less, and that the branched chain is a methyl group.

**16.** The method for producing the lubricating oil additive according to claim 13, comprising a step of allowing a fatty acid represented by the following general formula (11) and an amine compound represented by the following general formula (12) to react with each other, the amine compound being used in an amount of 1 mol or more based on 1 mol of the fatty acid:

$R^3$-COOH (11)

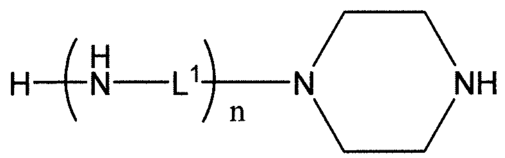 (12)

wherein $R^3$ is a straight-chain alkyl group having 8 to 22 carbon atoms, a branched-chain alkyl group having 8 to 22 carbon atoms, a straight-chain alkenyl group having 8 to 22 carbon atoms, or a branched-chain alkenyl group having 8 to 22 carbon atoms; $L^1$ is an alkylene group having 1 to 6 carbon atoms; and n is an integer of 1 to 4, provided that the number of branched chains which each of the branched-chain alkyl group and the branched-chain alkenyl group has is 3 or less, and that the branched chain is a methyl group.

## INTERNATIONAL SEARCH REPORT

International application No.
PCT/JP2015/068172

A. CLASSIFICATION OF SUBJECT MATTER
*C10M133/16*(2006.01)i, *C10N30/00*(2006.01)n, *C10N30/06*(2006.01)n, *C10N40/04*(2006.01)n

According to International Patent Classification (IPC) or to both national classification and IPC

B. FIELDS SEARCHED

Minimum documentation searched (classification system followed by classification symbols)
C10M133/16, C10N30/00, C10N30/06, C10N40/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 8-183979 A (Nippon Oil Co., Ltd.), 16 July 1996 (16.07.1996), claims; paragraphs [0054] to [0088]; examples (Family: none) | 1-5,8,11, 13-15 |
| X | JP 2-24395 A (Dai-Ichi Kogyo Seiyaku Co., Ltd.), 26 January 1990 (26.01.1990), claims; page 2, lower left column, line 5 to lower right column, line 8; examples (Family: none) | 1-5,8,11, 13-15 |
| X | JP 62-164796 A (Idemitsu Kosan Co., Ltd.), 21 July 1987 (21.07.1987), claims; page 2, lower left column, line 17 to page 3, lower left column, line 1; examples (Family: none) | 1-5,8,11, 13-15 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 July 2015 (22.07.15) | 04 August 2015 (04.08.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | <br>Telephone No. |

Form PCT/ISA/210 (second sheet) (July 2009)

**INTERNATIONAL SEARCH REPORT**

International application No.
PCT/JP2015/068172

C (Continuation). DOCUMENTS CONSIDERED TO BE RELEVANT

| Category* | Citation of document, with indication, where appropriate, of the relevant passages | Relevant to claim No. |
|---|---|---|
| A | JP 39-3115 B1 (California Research Corp.), 26 March 1964 (26.03.1964), entire text (Family: none) | 1-16 |
| A | US 2956020 A (Esso Standard Societe Anonyme Francaise), 11 October 1960 (11.10.1960), entire text & GB 782879 A & GB 810715 A & FR 1123818 A | 1-16 |
| A | JP 2008-88244 A (Idemitsu Kosan Co., Ltd.), 17 April 2008 (17.04.2008), entire text (Family: none) | 1-16 |
| A | JP 52-155605 A (Idemitsu Kosan Co., Ltd. et al.), 24 December 1977 (24.12.1977), entire text (Family: none) | 1-16 |

Form PCT/ISA/210 (continuation of second sheet) (July 2009)